# EUROPEAN PATENT APPLICATION

(11) **EP 4 682 898 A1**
(43) Date of publication of application: **21.01.2026**
(21) Application number: 24189411.2
(22) Date of filing: 18.07.2024
(51) Int. Cl.: G16H 20/00, G16H 50/20, A61B 5/00, A61B 5/20

(54) **MULTI-FACTOR RENAL DENERVATION INDEX FOR PATIENT SUITABILITY AND/OR EXPECTED RESPONSIVENESS TO RENAL DENERVATION TREATMENT**

(71) Applicant: Koninklijke Philips N.V., 5656 AG Eindhoven (NL)
(72) Inventor: CEZO, James David, Eindhoven (NL); HALILI, Reynaldo Borja Jr., Eindhoven (NL); CHEN, Sara Rose, 5656AG Eindhoven (NL)
(74) Representative: Philips Intellectual Property & Standards

(57) **Abstract**

An apparatus includes a processor configured to obtain values for a plurality of physiological metrics for a patient from at least one of an electronic health record database, a non-invasive measurement device, or an invasive measurement device. The processor is also configured to calculate a value of a renal denervation index using the physiological metrics such that the renal denervation index is itself not measured from the patient, where the value of the renal denervation index is representative of at least one of a suitability of the patient for a renal denervation treatment or an expected responsiveness of the patient to the renal denervation treatment. The processor is also configured to output, to a display, a screen display based on the value of the renal denervation index.

## Description

### FIELD

The subject matter described herein relates to systems, devices, and methods for determining the suitability and/or expected responsiveness of patients for a renal denervation (RDN) procedure. This RDN suitability prediction system has particular but not exclusive utility for identifying candidates who are likely to respond to a renal denervation procedure.

### BACKGROUND

Renal denervation is a potentially promising procedure for the treatment of hypertension. Recent studies have shown varying levels of efficacy with some portion of the population (currently estimated at approximately 30%) showing no response or negative response to the procedure. This demographic of non-responders may present as such for reasons that may stem from a combination of physiological factors and/or inadequately administered therapy. At present, there is no known parameter, algorithm, or method that adequately predicts treatment responders, nor whether a therapy has been applied effectively. Some efforts have been made to assess pulse-wave velocity (PWV) as an indicator.

The information included in this Background section of the specification, including any references cited herein and any description or discussion thereof, is included for technical reference purposes only and is not to be regarded as subject matter by which the scope of the disclosure is to be bound.

### SUMMARY

Disclosed is an RDN suitability prediction system. The present disclosure involves utilizing a collection of multiple physiological parameters/measurements as inputs to generated a single value or index that can be used to stratify patients and/or confirm completeness of therapy as an output. Input parameters may include combinations of patient data (e.g., weight/ BMI, other vitals) with real-time or point of care invasive or non-invasive measurements (e.g. pulse-wave velocity, vascular resistance, blood pressure, CT/angio-based physiology measurements, etc.). The RDN suitability prediction system disclosed herein has particular, but not exclusive, utility for determining the suitability of candidates for, and/or the completion/success of, renal denervation procedures.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the RDN suitability prediction system, as defined in the claims, is provided in the following written description of various aspects of the disclosure and illustrated in the accompanying drawings.

### BRIEF DESCRIPTION OF THE DRAWINGS

Illustrative aspects of the present disclosure will be described with reference to the accompanying drawings, of which:
**Figure** 1 is a schematic, diagrammatic representation, in block diagram form, of an example RDN suitability prediction system, in accordance with aspects of the present disclosure.
**Figure 2** is a schematic, diagrammatic representation, in block diagram form, of an example RDN index calculation process, according to aspects of the present disclosure.
**Figure 3** is a schematic, diagrammatic representation, in block diagram form, of an example RDN suitability and responsiveness calculation process, according to aspects of the present disclosure.
**Figure 4** is a screen display of an example RDN suitability prediction system, according to aspects of the present disclosure.
**Figure 5** is a schematic, diagrammatic representation, in block diagram form, of an example calculation process for the weights for an RDN index calculation, according to aspects of the present disclosure.
**Figure 6** is a schematic, diagrammatic representation, in block diagram form, of an example machine learning model training process for RDN index calculation, according to aspects of the present disclosure.
**Figure 7** is a schematic, diagrammatic representation, in block diagram form, of an example machine learning model training system for RDN index calculation, according to aspects of the present disclosure.
**Figure 8** is a schematic, diagrammatic representation, in block diagram form, of an example machine learning model inference mode for RDN index calculation, according to aspects of the present disclosure.
**Figure 9** is a schematic, diagrammatic representation, in block diagram form, of an example location-specific RDN index calculation process, according to aspects of the present disclosure.
**Figure 10** is a screen display of an example RDN suitability prediction system, according to aspects of the present disclosure.
**Figure 11** is a screen display of an example RDN suitability prediction system, according to aspects of the present disclosure.
**Figure 12** is a schematic, diagrammatic representation, in block diagram form, of an example time-specific RDN index calculation process, according to aspects of the present disclosure.
**Figure 13** is a screen display of an example RDN suitability prediction system, according to aspects of the present disclosure.
**Figure 14** is a schematic diagram of a processor circuit, according to aspects of the present disclosure.
**Figure 15** is a schematic, diagrammatic representation of the renal vasculature of a patient, with a renal denervation treatment device in the left renal artery of the left kidney, according to aspects of the present disclosure.

### DETAILED DESCRIPTION

In accordance with at least one aspect of the present disclosure, a RDN suitability prediction system is provided which enables a clinician to predict which candidates are likely to show favorable response to RDN treatment, and/or to determine the success or completion of an RDN treatment in progress.

The present disclosure involves utilizing a collection of multiple physiological parameters/measurements as inputs. The output includes a single value or index that can be used to stratify patients and/or confirm completeness of therapy. Input parameters may include combinations of patient data (e.g. weight/ BMI, other vitals) with real-time or point of care invasive or non-invasive measurements (e.g. pulse-wave velocity, vascular resistance, blood pressure, CT/angio-based physiology measurements, etc.).

A key aspect of the present disclosure is the utilization of more than one physiologic metric to create an index or RDN stratification score. The measurements may consist of physiologic metrics taken at a single time point, their time histories, or their change over time. The metrics which are inputs to may include one or several of the following measures, height, weight, body-mass index (BMI), blood pressure (BP), ambulatory BP, mean BP, systolic BP, diastolic BP, heart rate (HR), resting HR, peak HR, mean HR, imaging-derived (e.g., X-ray, angiography, computer-aided tomography (CT) scan, positron emission tomography (PET) scan, magnetic resonance imaging (MRI), or ultrasound-derived) measurements, or invasive measurement such as pulse-wave velocity, vascular resistance, renal vascular resistance, vascular flow rate, microvascular resistance, nerve conduction, renal nerve resting potential, and chemical assays for blood or urine to detect levels of biomarker chemicals such as creatinine, urea, cystatin, angiotensin, and/or renin. Inputs may also include medical metrics for assessing health of the heart, cardiovascular system, or kidneys such as glomerular filtration rate (GFR), aortic stiffness index (ASI), and acute kidney injury classification (AKI).

An index is calculated/determined using these inputs. The index can score the patient according to the impact that a renal denervation procedure will have to their blood pressure. The index calculation/determination may be derived from a set of pre-procedure patient data paired to outcomes post-RDN. For example, a predictive network (e.g., a machine learning network, artificial intelligence network, etc.) may be trained using such a dataset to identify the input parameters with best correlation to post-RDN outcomes such as BP reduction.

In an alternative embodiment the inputs are multiple parameters taken pre-RDN and post-RDN treatment to assess the success of the procedure.

The present disclosure aids substantially in performing interventional procedures such as renal denervation, by improving the clinician's ability to understand and measure the expected effectiveness of the treatment. Implemented on a processor in communication with one or more sensors or databases, the RDN suitability prediction system disclosed herein provides practical detection and measurement of the patient's expected response to the RDN treatment. This improved situational awareness transforms a blind medical procedure with a 70% success rate into one where the success can be accurately predicted and even measured in real time, to determine if more treatment is necessary, without the normally routine need to wait and see whether the patient's hypertension declines over period of days or weeks. This unconventional approach improves the functioning of the renal denervation system, by improving the success rate of RDN treatments and by screening out candidates who are not suitable for the procedure.

The RDN suitability prediction system may be implemented at least partially as a process viewable on a display, and operated by a control process executing on a processor that accepts user inputs from a keyboard, mouse, or touchscreen interface, and that is in communication with one or more sensors. In that regard, the control process performs certain specific operations in response to different inputs or selections made at different times. Certain outputs of the RDN suitability prediction system may be printed, shown on a display, indicated with lights or tones, or otherwise communicated to human operators. Certain structures, functions, and operations of the processor, display, sensors, and user input systems are known in the art, while others are recited herein to enable novel features or aspects of the present disclosure with particularity.

The present disclosure is related to U.S. Provisional Application No. ___, filed ___, and titled "Catheter-Based Procedures With Procedure Room Detection Of Biomarkers In Patient Blood And Associated Devices, Systems, And Methods" (Atty. Dkt. No. 2023PF00849 / 44755.2399PV01), U.S. Provisional Application No. ___, filed ___, and titled "Renal Denervation Treatment Guidance Using Hemodynamic Co-Registration and Associated Systems, Devices, Methods" (Atty. Dkt. No. 2023PF00857 / 44755.2405PV01), U.S. Provisional Application No. ___, filed ___, and titled "Renal Nerve Bundle Co-Registration With X-Ray Image For Renal Denervation Treatment Guidance" (Atty. Dkt. No. 2023PF00858 / 44755.2406PV01), and U.S. Provisional Application No. ___, filed ___, and titled "Renal Denervation Treatment Assessment Using Ambulatory Blood Pressure Monitor" (Atty. Dkt. No. 2024PF00043 / 44755.2414PV01), each of which is incorporated by reference as though fully set forth herein.

These descriptions are provided for exemplary purposes only, and should not be considered to limit the scope of the RDN suitability prediction system. Certain features may be added, removed, or modified without departing from the spirit of the claimed subject matter.

For the purposes of promoting an understanding of the principles of the present disclosure, reference will now be made to the aspects illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one aspect may be combined with the features, components, and/or steps described with respect to other aspects of the present disclosure. For the sake of brevity, however, the numerous iterations of these combinations will not be described separately.

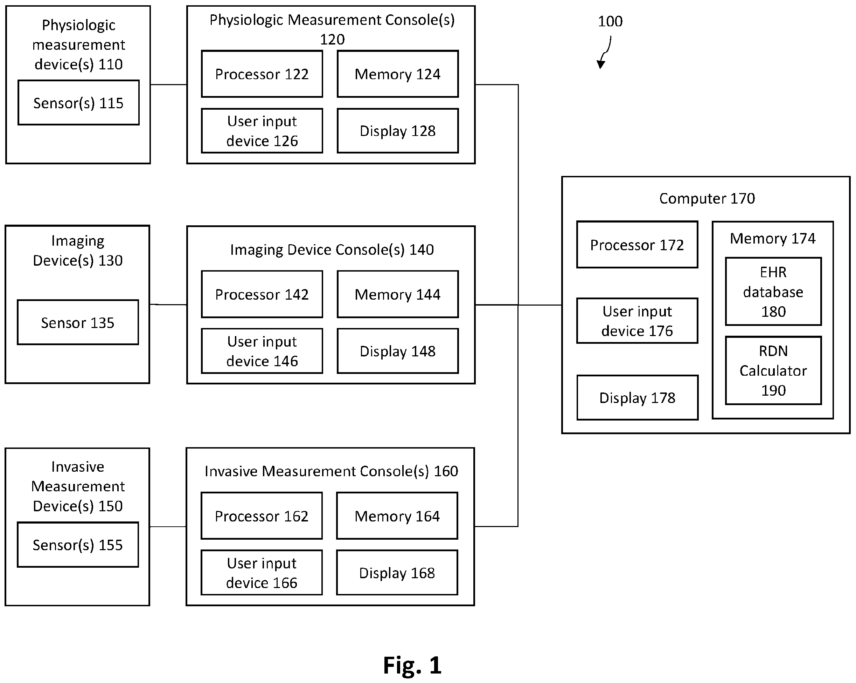

**Figure 1** is a schematic, diagrammatic representation, in block diagram form, of an example RDN suitability prediction system 100, according to aspects of the present disclosure. The RDN suitability prediction system can include one or more physiologic measurement devices 110, one or more imaging devices 130, and one or more invasive measurement devices 150.

In an example, the physiologic measurement device 110 includes one or more sensors 115, and is in communication with one or more physiologic measurement consoles 120. A physiologic measurement console 120 may for example include a processor 122, memory 124, user input device 126, and display 128. In some aspects, the physiologic measurement device 110 may not require a physiologic measurement console 120. In other aspects, the physiologic measurement console 120 may be integrated into the physiologic measurement device 110. In some cases, a single physiologic measurement console 120 can support multiple physiologic measurement devices 110. In other cases, a single physiologic measurement device 110 may communicate with multiple physiologic measurement consoles 120. Physiologic measurements may for example include, but are not limited to, blood pressure (BP), ambulatory BP, mean BP, systolic BP, diastolic BP, heart rate (HR), resting HR, peak HR, mean HR, etc. The physiologic measurement device 110 may for example be a blood pressure cuff, a pulse oximeter, an electrocardiogram (EKG) machine, etc.

In an example, the imaging device 130 includes one or more imaging sensors 135, and is in communication with one or more physiologic measurement consoles 120. An imaging device console 140 may for example include a processor 142, memory 144, user input device 146, and display 148. In some aspects, the imaging device console 140 may be integrated into the imaging device 130. In some cases, a single imaging device console 140 can support multiple imaging devices 130. Imaging devices may for example include, but are not limited to, X-ray, angiography, CT scan, PET scan, MRI, or ultrasound. Certain imaging-derived measurements may be determined by the console 140 and/or the computer 170, including but not limited to blood flow velocity, pulse wave velocity, volumetric flow rate, vessel contractility, arterial stiffness, and vessel/artery diameter changes over time.

In an example, the invasive measurement device 150 includes one or more sensors 155, and is in communication with one or more invasive measurement consoles 160. An invasive measurement console 160 may for example include a processor 162, memory 164, user input device 166, and display 168. In some aspects, the invasive measurement device 150 may not require an invasive measurement console 160. In other aspects, the invasive measurement console 160 may be integrated into the invasive measurement device 150. In some cases, a single invasive measurement console 160 can support multiple invasive measurement devices 150. Invasive measurements may for example include, but are not limited to, mean BP, systolic BP, diastolic BP, blood flow velocity, intravascular ultrasound (IVUS) imaging, pulse-wave velocity, vascular resistance, renal vascular resistance, vascular flow rate, microvascular resistance, nerve conduction, renal nerve resting potential, and concentrations of biomarkers in the blood or other body fluids. Invasive measurement devices include intravascular or intraluminal pressure-sensing, flow-sensing, and intravascular ultrasound (IVUS) imaging catheters and guidewires.

In an example, the computer 170 may include a processor 172, memory 174, user input device 176, and display 178. The memory 174 may include an electronic health records database 180, which includes patient data including but not limited to vital signs, demographics, past imaging and physiologic measurements, etc. The memory 174 may also include an RDN calculator 190, the operation of which will be described below. The EHR database 180 and RDN calculator 190 can be part of different computers. For example, the EHR database 180 can be part of a hospital computer or server, whereas the RDN calculator 190 can be on a network computer, cloud computer, or server, which is in communication with hospital computer or server.

Block diagrams are provided herein for exemplary purposes; a person of ordinary skill in the art will recognize myriad variations that nonetheless fall within the scope of the present disclosure. For example, any of the steps described herein may optionally include an output to a user of information relevant to the step, and may thus represent an improvement in the user interface over existing art by providing information not otherwise available to the user. Similarly, block diagrams may show a particular arrangement of components, modules, services, steps, processes, or layers, resulting in a particular data flow. It is understood that some aspects of the systems disclosed herein may include additional components, that some components shown may be absent from some aspects, and that the arrangement of components may be different than shown, resulting in different data flows while still performing the methods described herein.

Before continuing, it should be noted that the examples described above are provided for purposes of illustration, and are not intended to be limiting. Other devices and/or device configurations may be utilized to carry out the operations described herein.

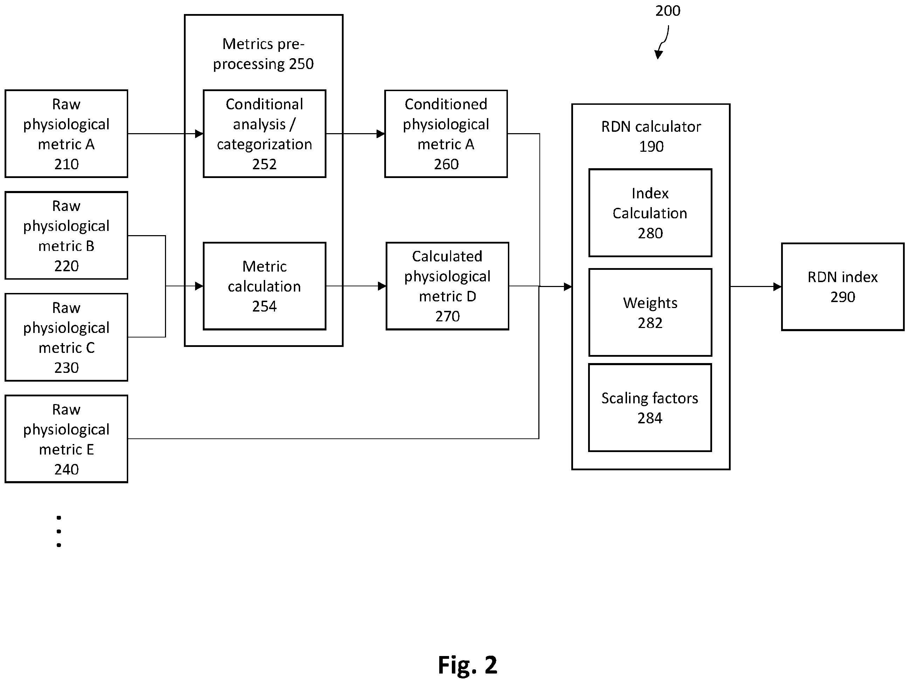

**Figure 2** is a schematic, diagrammatic representation, in block diagram form, of an example RDN index calculation process 200, according to aspects of the present disclosure. The calculation process 200 begins with raw physiological metrics 210, 220, 230, 240, etc. The raw physiologic metrics may for example include invasive and/or non-invasive measurements or images as described above. In the example shown in Figure 2, raw physiological metric E, 240, is passed directly to the RDN calculator 190. Depending on the implementation, some, all, or none of the raw physiological metrics may be used in this way.

Similarly, in the example shown in Figure 2, raw physiological metric A, 210, is passed to a metrics pre-processing module 250 which includes a conditional analysis and/or categorization module 252. For example, a parameter such as BMI, instead of being used in its raw form, may be conditionally sorted into one of three categories (e.g., 1 for BMI values of 25 or less, 2 for BMI values between 25 and 30, and 3 for BMI values of 30 or more). Other examples of conditional sorting, analysis, or categorization, as would occur to a person of ordinary skill in the art, may be used instead or in addition. This conditioned physiological metric 260 is then passed to the RDN calculator 190.

Some physiological metrics can be calculated from two or more raw measurements. In the example shown in Figure 2, raw physiological metrics 220 and 230 (e.g., pressure, flow, imaging, etc.) are used in a metric calculation 254 of the pre-processing module 250, to produce a calculated physiological metric 270, which is then passed to the RDN calculator 190. Examples of calculated physiological metrics include, but are not limited to, pulse wave velocity (PWV), mean blood pressure, blood flow velocity, vascular resistance or impedance, arterial stiffness, artery contractility, and renal resistance. Examples of pulse wave velocity calculation can be found in U.S. Publication No. 2019/0290139, filed May 19, 2017, U.S. Publication No. 20190175035, filed May 19, 2017, U.S. Patent No. 11,896,422, filed August 13, 2019, and U.S. Publication No. 20230233168, filed January 19, 2023, each of which is incorporated by reference as though fully set forth herein.

The RDN calculator 190 includes an index calculation 280. In the example shown in Figure 2, the inputs to the index calculation 280 are conditioned physiological metric 260, calculated physiological metric 270, and raw physiological metric 240, although it is understood that other input metrics or combinations of input metrics may be used instead or in addition. In some aspects, the index calculation may be a simple arithmetic combination of the input metrics, such as a sum or product. In other aspects, the index calculation 280 may rely on weights 282 and/or scaling factors 284 that are applied to the input metrics. For example, the weights 282 may quantify the relative importance of different input metrics such that, for example, PWV (with an exemplary weight of 4.0) may be twice as important as mean blood pressure (with an exemplary weight of 2.0) and four times as important as BMI (with a weight of 1.0) for the accurate prediction of a patient's response to the RDN procedure. Scaling factors 284 may be used for example to combine input metrics having different scale, so that, for example, BMI (generally within the range of 12-50) may be multiplied by 1/3 to scale it proportionally to pulse wave velocity (with typical values between 6 m/s and 20 m/s. In another example, BMI (with a conditioned value of 1, 2, or 3) may be multiplied by 5.0 to scale it proportionally to pulse wave velocity (with typical values between 6 m/s and 20 m/s). Weights 282 and scaling factors 284 may for example be developed based on clinical research, medical literature, standards set by physician's organizations, and otherwise. In some cases, for negative correlations, a weight or scaling factor may be less than zero.

The output of the index calculation 280 is a single RDN index 290, which may for example be a value between 0.0 and 1.0, a value between 0 and 100, or a value with no specific upper or lower bounds, such as a predicted amount of systolic blood pressure reduction (e.g., in mmHg), that is nevertheless representative of the patient's suitability for, and probable response to, the RDN procedure. Since the RDN depends on at least two raw physiological metrics, the RDN calculator does not report an RDN index that is equal to any one raw physiological metric.

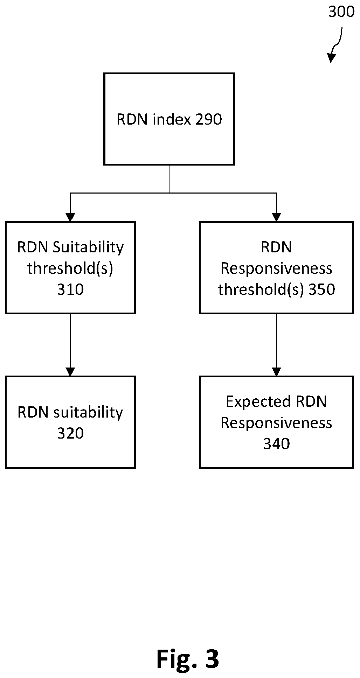

**Figure 3** is a schematic, diagrammatic representation, in block diagram form, of an example RDN suitability and responsiveness calculation process 300, according to aspects of the present disclosure. In the example shown in Figure 3, the calculated RDN index 290 for a patient is compared against one or more RDN suitability thresholds 310 to produce an RDN suitability 320. In a non-limiting example, for an RDN index 290 between 0 and 100, a threshold 310 of 60 or above may indicate high suitability, while a threshold 310 of 40 or above (e.g., a value between 40 and 60) may indicate medium suitability, and values below the threshold of 40 may indicate low suitability.

Similarly, the RDN index 290 may be compared against one or more responsiveness thresholds 350 to yield an expected RDN responsiveness 340. In a non-limiting example, an RDN index value below a threshold of 60 may indicate low responsiveness (e.g., an expected blood pressure reduction of 0-2 mmHg), whereas an RDN index value between the thresholds of 60 and 75 may indicate medium responsiveness (e.g., an expected blood pressure reduction of 2-5 mmHg), and an RDN index between the thresholds of 75 and 100 may indicate high responsiveness (e.g., an expected blood pressure reduction of greater than 5 mmHg).

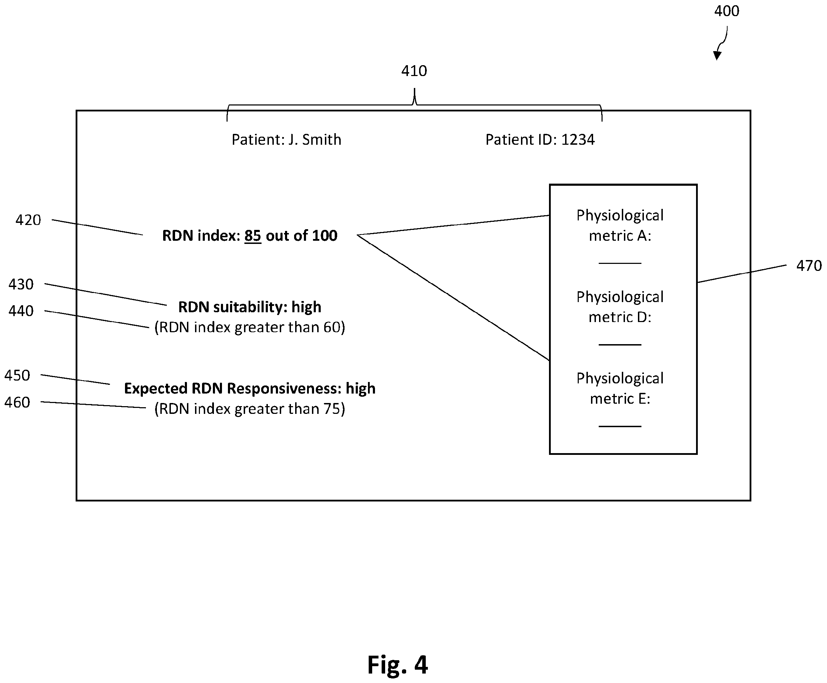

**Figure 4** is a screen display 400 of an example RDN suitability prediction system, according to aspects of the present disclosure. The screen display 400 includes patient information 410, an RDN index display 420, an RDN suitability display 430 with explanation 440, and an expected RDN responsiveness display 450 with explanation 460. In the example shown in Figure 4, to help clinicians understand the derivation and meaning of the RDN index, the screen display 400 also includes a physiological measurements window 470, showing the raw measurement values and/or computed metrics that were used to compute the RDN index, as described above.

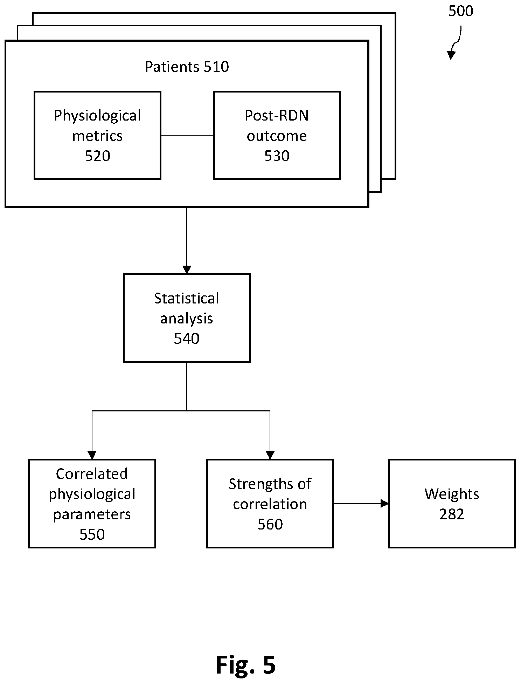

**Figure 5** is a schematic, diagrammatic representation, in block diagram form, of an example calculation process 500 for the weights 282 for an RDN index calculation, according to aspects of the present disclosure. The calculation process 500 begins with physiological measurements 520 and post-RDN outcomes 530 (e.g., measured systolic blood pressure reduction) for a population of patients 510. These values 520, 520 serve as inputs to a statistical analysis 540 that yields a list of correlated physiological parameters 550, e.g., a list of those physiological metrics 520 that show a statistically significant correlation with the post-RDN outcomes 530. Statistical analysis may for example include linear or non-linear regression of multiple independent variables in search of the strongest correlation to the post-RDN outcomes 530. The statistical analysis also yields the strengths 560 of these correlations, which can then either serve as weights 282 or can be used to calculate the weights 282.

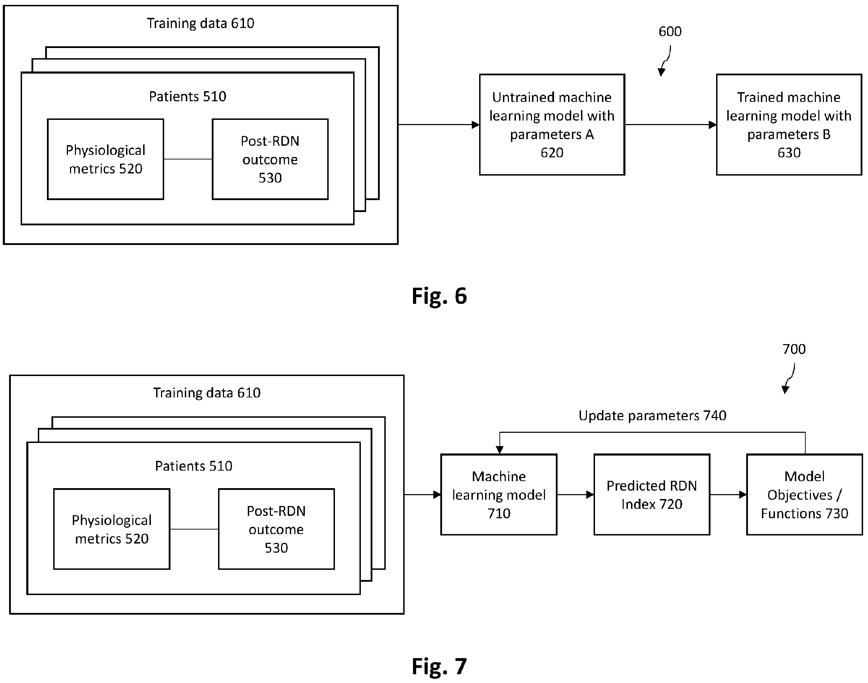

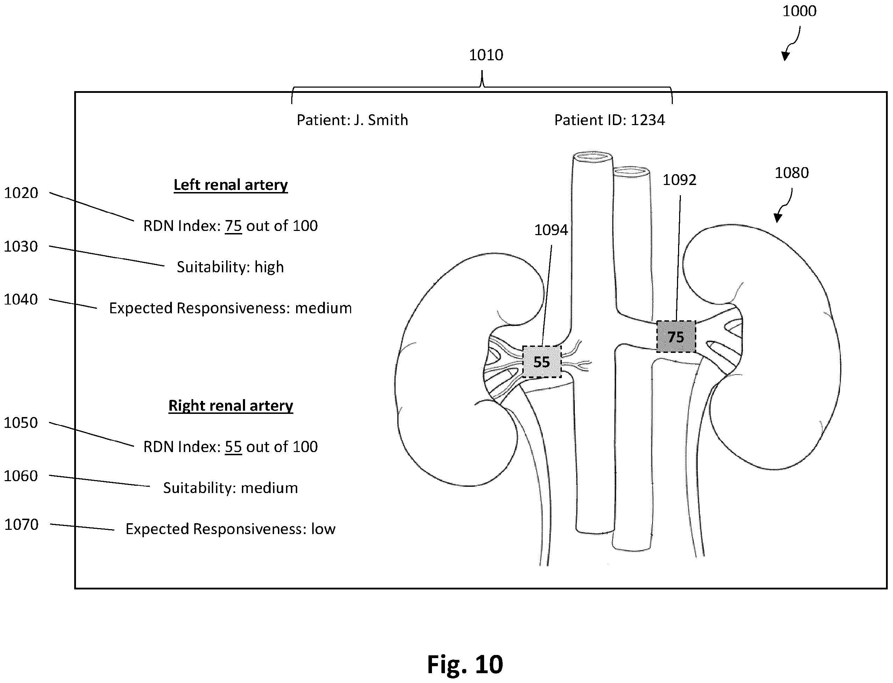

**Figure 6** is a schematic, diagrammatic representation, in block diagram form, of an example machine learning model training process 600 for RDN index calculation, according to aspects of the present disclosure. The training process 600 begins with a set of training data 610 (e.g., supervised learning data) that includes physiological measurements 520 and post-RDN outcomes 530 (e.g., measured systolic blood pressure reduction) for a population of patients 510. These values 520, 520 are used to train an untrained machine learning model 620 with parameters A, to produce a trained machine learning model 630 with parameters B, that can be used in inference mode to calculate an RDN index for a patient based on that patient's physiological metrics. In an example, the machine learning model uses supervised learning based on regression models or tree-based models. In an example, regression model(s) can be used (as opposed to classification model(s)), such as if continuous index is used (e.g., Figure 10). Continuous and/or non-continuous (e.g., discrete, categorized) indices are contemplated.

**Figure 7** is a schematic, diagrammatic representation, in block diagram form, of an example machine learning model training system 700 for RDN index calculation, according to aspects of the present disclosure. The training 700 begins with a set of training data 610 that includes physiological measurements 520 and post-RDN outcomes 530 (e.g., measured systolic blood pressure reduction) for a population of patients 510. These values 520, 520 are used to train a predictive machine learning network or model 710, which produces predicted RDN indices 720 for each patient 510. These indices 720 are then evaluated against the model's objectives or functions 730 (e.g., a difference between the post-RDN outcome 530 and the expected post-RDN outcome associated with the predicted RDN index 720) to determine the success of the training, and, if the predicted RDN indices 720 fall below a threshold of desired accuracy (e.g. for predicting the post-RDN outcomes 530), the parameters go through repeated updates 740 until the desired accuracy is achieved. In some instances, updating 740 may be accomplished using gradients of the objective functions and backpropagation to update the parameters of the predictive network 710. In some embodiments, retraining and/or fine tuning can be done for newly introduced patient data (e.g., within clinical trials).

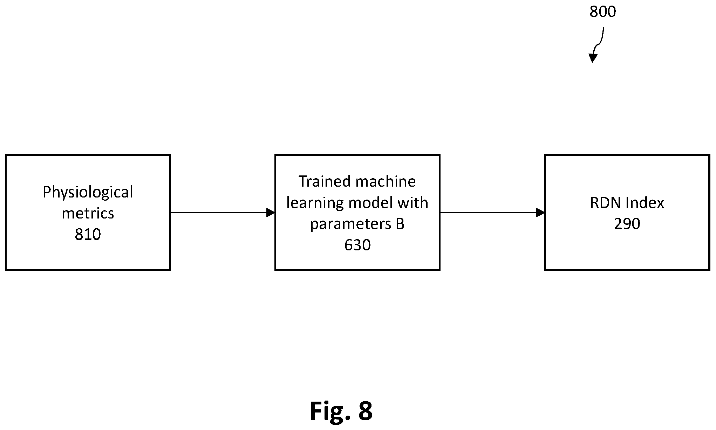

**Figure 8** is a schematic, diagrammatic representation, in block diagram form, of an example machine learning model inference mode 800 for RDN index calculation, according to aspects of the present disclosure. When the trained machine learning model 630 receives physiological metrics 810 for a new patient, it produces an RDN index 290 indicative of the likelihood of success of an RDN treatment for that patient, and/or a predicted level of response (e.g., a predicted systolic blood pressure reduction). This may for example provide a clinician with the information needed to determine whether to perform an RDN procedure on the patient.

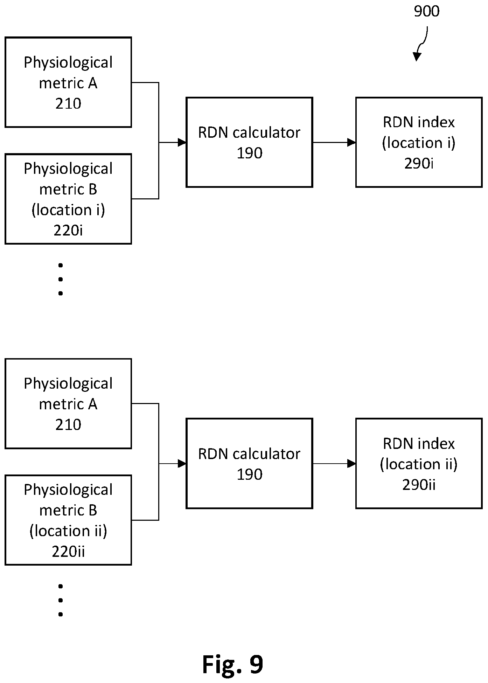

**Figure 9** is a schematic, diagrammatic representation, in block diagram form, of an example location-specific RDN index calculation process 900, according to aspects of the present disclosure. In the example shown in Figure 9, a first physiological metric A, 210 (e.g., BMI or patient age) may be insensitive or relatively insensitive to a measurement location, whereas a second physiological metric B, 220 (e.g., blood flow velocity) may vary with the measurement location, such that at a location i, the second physiological metric B has a value 220i, and at a location ii, the second physiological metric B has a value 220ii. Thus, when the metrics 210 and 220i from location i are fed into the RDN calculator 190, the RDN calculator 190 produces an RDN index 290i for location i. Similarly, when the metrics 210 and 220ii from location ii are fed into the RDN calculator 190, the RDN calculator 190 produces an RDN index 290ii for location ii. The location-specific differences in these values may have clinical significance, such as for example the RDN index being different for the left renal artery than for the right renal artery, or being different for two different locations in the left or right renal artery, as described below.

**Figure 10** is a screen display 1000 of an example RDN suitability prediction system, according to aspects of the present disclosure. The screen display 1000 includes patient information 1010, as well as a left renal artery RDN index 1020, suitability 1030, and expected responsiveness 1040, and a right renal artery RDN index 1050, suitability 1060, and expected responsiveness 1070. In the example shown in Figure 10, the screen display 1000 also includes a stylized representation 1080 of kidney anatomy, along with color-coded RDN index tags 1092, 1094 for the left and right renal arteries, respectively. As can be seen in the figure, the left renal artery RDN index 1020 has a value of 75 out of 100, indicated with a dark gray in tag 1092, and representing a high suitability 1030 and medium expected responsiveness 1040 for the RDN procedure. Conversely, the right renal artery RDN index 1050 has a value of 55 out of 100, indicated with a lighter gray in tag 1904, and representing a medium suitability 1060 and low expected responsiveness 1070. Thus, a clinician viewing the screen display 1000 may conclude that RDN treatment is desirable for the left renal artery and not desirable for the right renal artery. Color coding of the tags 1092, 1094 may be brighter for higher RDN index values and darker for lower RDN values, or may be red/yellow/green depending on the suitability, or may use other color schemes as would occur to a person of ordinary skill in the art.

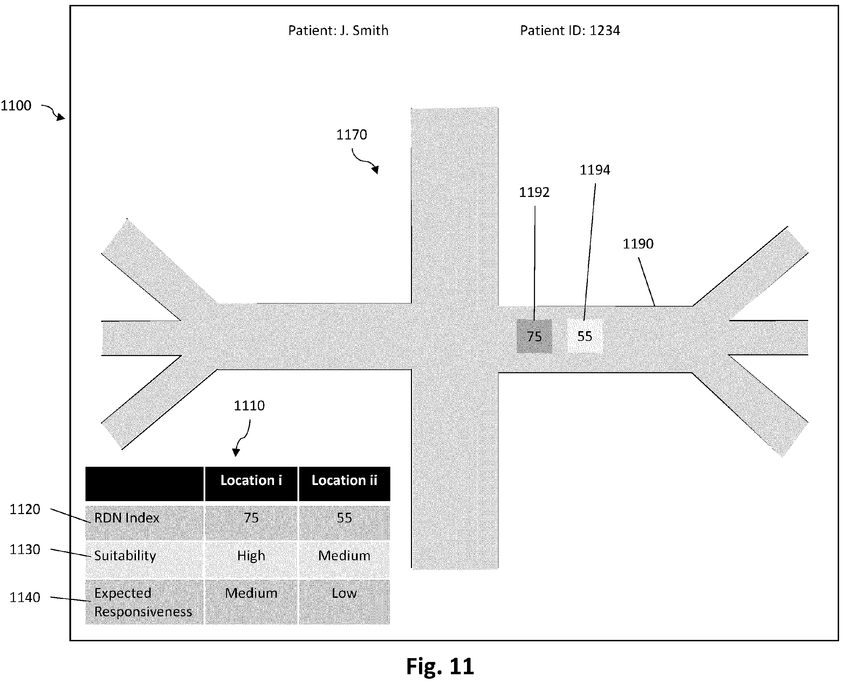

**Figure 11** is a screen display 1100 of an example RDN suitability prediction system, according to aspects of the present disclosure. The screen display 1100 includes an x-ray image with contrast (e.g., angiogram) of the renal arterial tree 1170 including the left renal artery 1190. The screen display 1100 also includes a table 1110 showing RDN index 1120, suitability 1130, and expected responsiveness 1140 for two different locations, i and ii, within the left renal artery, also indicated by color-coded tags 1192 and 1194, respectively. At location i, the RDN index 1120 has a value of 75, associated with a high suitability 1130 and a medium expected responsiveness 1140, indicated by a dark gray color in tag 1192. Conversely, at location ii, the RDN Index has a value of 55, associated with a medium suitability 1130 and a low expected responsiveness 1140, indicated by a light gray color in tag 1194. Thus, a clinician viewing the screen display 1100 may conclude that RDN treatment is desirable for location i within the left renal artery, but not desirable for location ii within the left renal artery. Displaying the color-coded tags 1192 and 1194 at corresponding locations of the left renal artery 1190 can be based on co-registration. Aspects of co-registration are described for example in U.S. Patent No. 7,930,014, titled "Vascular image co-registration", and U.S. Publication No. 2012/0004537, titled "Co-use of endoluminal data and extraluminal imaging", each of which is incorporated by reference as though fully set forth herein.

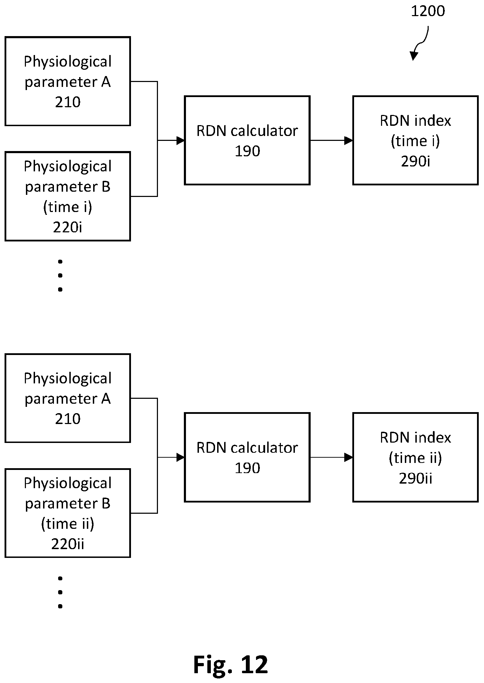

**Figure 12** is a schematic, diagrammatic representation, in block diagram form, of an example time-specific RDN index calculation process 1200, according to aspects of the present disclosure. In the example shown in Figure 12, a first physiological metric A, 210 (e.g., BMI or patient age) may be insensitive or relatively insensitive to a measurement time, whereas a second physiological metric B, 220 (e.g., pulse wave velocity) may vary with the measurement time (especially during the RDN treatment itself), such that at a time i, the second physiological metric B has a value 220i, and at a time ii, the second physiological metric B has a value 220ii. Thus, when the metrics 210 and 220i from time i are fed into the RDN calculator 190, the RDN calculator 190 produces an RDN index 290i for time i. Similarly, when the metrics 210 and 220ii from time ii are fed into the RDN calculator 190, the RDN calculator 190 produces an RDN index 290ii for time ii. The time-specific differences in these values may have clinical significance, such as for determining the progress and/or completeness of an RDN procedure while the procedure is taking place, as described below.

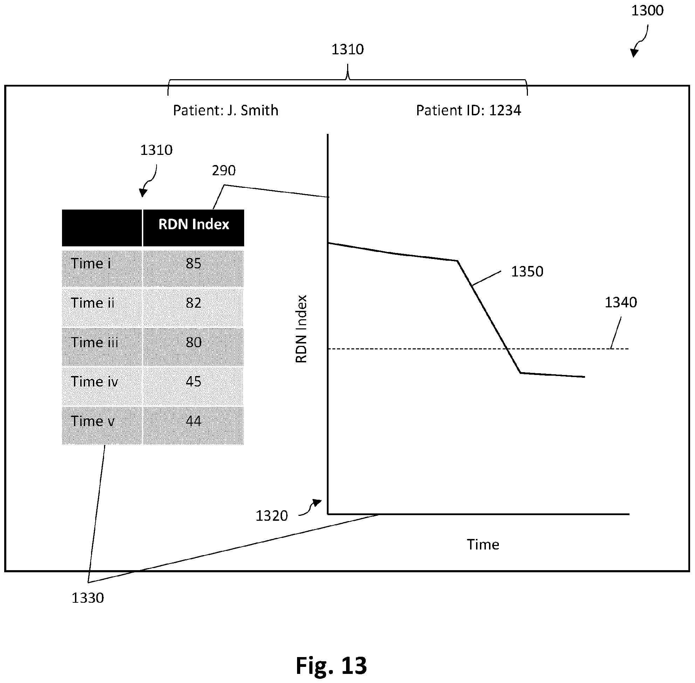

**Figure 13** is a screen display 1100 of an example RDN suitability prediction system, according to aspects of the present disclosure. The screen display 1100 includes a table 1310 and a graph 1320 showing RDN index 290 vs. time 1330. The graph 1320 includes a threshold value 1340 and a progress curve 1350. The progress curve 1350 shows the change in RDN index 290 over time, and in the example shown in Figure 13, the RDN index 290 declines while treatment is being performed. Once the RDN index 290 drops below the threshold 1340, the treatment may be considered complete and/or successful.

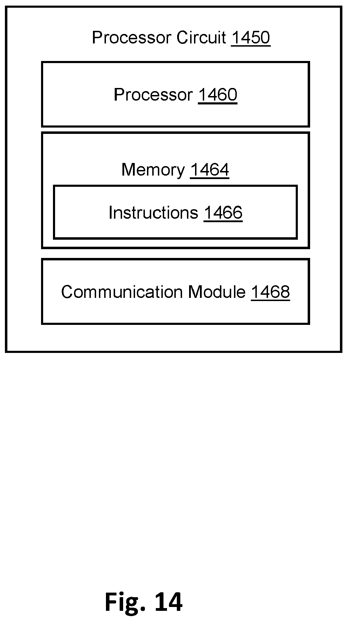

**Figure 14** is a schematic diagram of a processor circuit 1450, according to aspects of the present disclosure. The processor circuit 1450 may be implemented in system 100, processor 122, processor 142, processor 162, processor 172, or other devices or workstations (e.g., third-party workstations, network routers, etc.), or on a cloud processor or other remote processing unit, as necessary to implement the method. As shown, the processor circuit 1450 may include a processor 1460, a memory 1464, and a communication module 1468. These elements may be in direct or indirect communication with each other, for example via one or more buses.

The processor 1460 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1460 may also comprise another hardware device, a firmware device, or any combination thereof configured to perform the operations described herein. The processor 1460 may also be implemented as a combination of computing devices, e.g., a combination of a DSP and a microprocessor, a plurality of microprocessors, one or more microprocessors in conjunction with a DSP core, or any other such configuration.

The memory 1464 may include a cache memory (e.g., a cache memory of the processor 1460), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an aspect, the memory 1464 includes a non-transitory computer-readable medium. The memory 1464 may store instructions 1466. The instructions 1466 may include instructions that, when executed by the processor 1460, cause the processor 1460 to perform the operations described herein. Instructions 1466 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1468 can include any electronic circuitry and/or logic circuitry to facilitate direct or indirect communication of data between the processor circuit 1450, and other processors or devices. In that regard, the communication module 1468 can be an input/output (I/O) device. In some instances, the communication module 1468 facilitates direct or indirect communication between various elements of the processor circuit 1450 and/or the system 100. The communication module 1468 may communicate within the processor circuit 1450 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, data sharing between the processor and central server, or readings from the sensors 115, 135, 155) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles) , 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

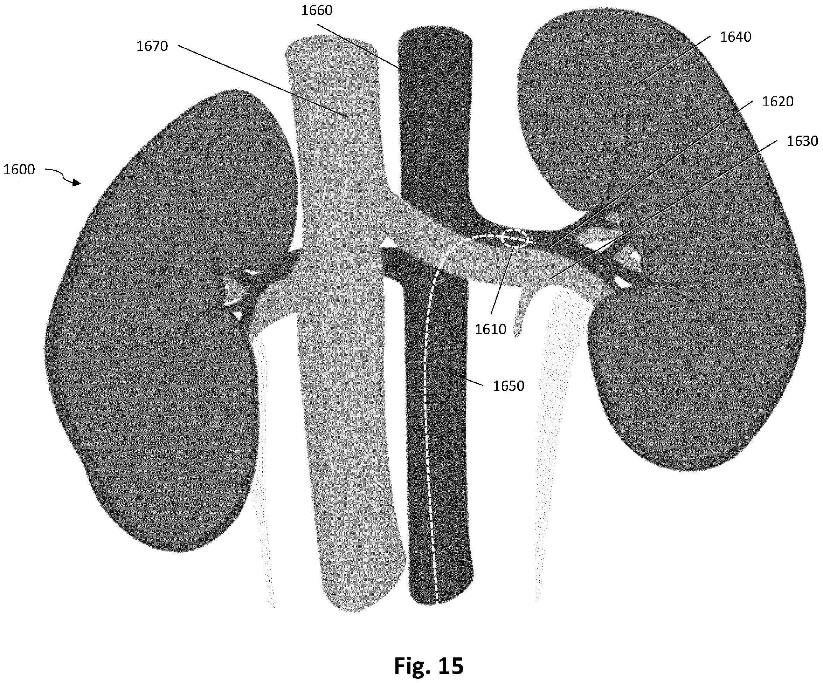

**Figure 15** is a schematic, diagrammatic representation of the renal vasculature 1600 of a patient, with a renal denervation treatment device 1610 in the left renal artery 1620 of the left kidney 1640, according to aspects of the present disclosure. In an example, the renal denervation treatment device 1610 may include a catheter 1650 with a renal denervation tool (e.g., electrodes, balloon, etc.) that delivers energy to injure the renal nerves, in order to lower the patient's blood pressure. Depending on the implementation, the delivered energy may be electrical energy, chemical energy, heat, cold, etc. The renal denervation treatment device 1610 may for example enter the renal artery 1620 via the abdominal aorta 1660. Also visible is the renal vein 1630. The renal denervation can be performed based on the guidance described herein.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes an apparatus. The apparatus includes a processor configured to: obtain values for a plurality of physiological metrics for a patient from at least one of an electronic health record database, a non-invasive measurement device, or an invasive measurement device; calculate a value of a renal denervation index using the plurality of physiological metrics such that the renal denervation index is itself not measured from the patient, where the value of the renal denervation index is representative of at least one of a suitability of the patient for a renal denervation treatment or an expected responsiveness of the patient to the renal denervation treatment; and output, to a display, a screen display based on the value of the renal denervation index. Other examples of this aspect include corresponding computer systems, apparatus, and computer programs recorded on one or more computer storage devices, each configured to perform the actions of the methods.

Implementations may include one or more of the following features. In some aspects, the renal denervation index may include a scale between a lowest value and a highest value. In some aspects, calculating the value of the renal denervation index using the plurality of physiological metrics involves applying at least one of weights or scaling factors to the physiological metrics. In some aspects, calculating the value of the renal denervation index using the plurality of physiological metrics involves applying conditional categorization to at least some of the physiological metrics. In some aspects, calculating the value of the renal denervation index using the plurality of physiological metrics involves at least one physiological metric whose value varies with measurement location, such that the renal denervation index varies with the measurement location. In some aspects, the varying of the renal denervation index with location is used to select a treatment location. In some aspects, the varying of the renal denervation index with time is used to detect progress of a renal denervation treatment. In some aspects, calculating the value of the renal denervation index using the plurality of physiological metrics involves at least one physiological metric whose value varies with measurement time, such that the renal denervation index varies with the measurement time. In some aspects, calculating the value of the renal denervation index using the plurality of physiological metrics involves calculating at least one physiological metrics using at least two other physiological metrics of the plurality of physiological metrics. In some aspects, calculating the value of the renal denervation index using the plurality of physiological metrics involves a trained machine learning network. In some aspects, the plurality of physiological metrics includes at least one of imaging-derived measurements or invasive measurements. In some aspects, the plurality of physiological metrics includes at least one of height, weight, body-mass index (BMI), blood pressure (BP), ambulatory BP, mean BP, systolic BP, diastolic BP, heart rate (HR), resting HR, peak HR, mean HR, pulse-wave velocity, vascular resistance, renal vascular resistance, vascular flow rate, microvascular resistance, nerve conduction, renal nerve resting potential, glomerular filtration rate (GFR), aortic stiffness index (ASI), acute kidney injury classification (AKI, or concentrations of a biomarker molecule. In some aspects, the non-invasive measurement device may include an x-ray, angiography, computer-aided tomography (CT), positron emission tomography (PET), magnetic resonance imaging (MRI), or ultrasound imaging device. In some aspects, the non-invasive measurement device may include a blood pressure cuff, a pulse oximeter, an electrocardiogram (EKG) machine. In some aspects, The invasive measurement device may include an intraluminal catheter or guidewire. Implementations of the described techniques may include hardware, a method or process, or computer software on a computer-accessible medium.

One general aspect includes a method. The method includes obtaining, with a processor, values for a plurality of physiological metrics for a patient from at least one of an electronic health record database, a non-invasive measurement device, or an invasive measurement device; calculating, with the processor, a value of a renal denervation index using the plurality of physiological metrics such that the renal denervation index is itself not measured from the patient, where the value of the renal denervation index is representative of at least one of a suitability of the patient for a renal denervation treatment or an expected responsiveness of the patient to the renal denervation treatment; and outputting, to a display, a screen display based on the value of the renal denervation index. Other examples of this aspect include corresponding computer systems, apparatus, and computer programs recorded on one or more computer storage devices, each configured to perform the actions of the methods.

Accordingly, it can be seen that the RDN suitability prediction system advantageously provides a means for determining, both in real time during a procedure and in near-real-time in advance of a procedure, a patient's suitability for, and likely response to, a renal denervation procedure.

A number of variations are possible on the examples and aspects described above. For example, other variables may be used than those listed herein, and other sensors or sensor types may be employed. The technology described herein may be used not only before and during medical interventions, but also at other times, to provide metrics that may be indicative of a health state or disease state of the patient..

Accordingly, the logical operations making up the aspects of the technology described herein are referred to variously as operations, steps, objects, elements, components, or modules. Furthermore, it should be understood that these may occur, or be performed or arranged, in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the RDN suitability prediction system. Connection references, e.g., attached, coupled, connected, joined, or "in communication with" are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the RDN suitability prediction system as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

Additional embodiments
Embodiment 1. An apparatus, comprising:
   a processor configured to:
   obtain values for a plurality of physiological metrics for a patient from at least one of an electronic health record database, a non-invasive measurement device, or an invasive measurement device;
   calculate a value of a renal denervation index using the plurality of physiological metrics such that the renal denervation index is itself not measured from the patient, wherein the value of the renal denervation index is representative of at least one of a suitability of the patient for a renal denervation treatment or an expected responsiveness of the patient to the renal denervation treatment; and
   output, to a display, a screen display based on the value of the renal denervation index.
Embodiment 2. The apparatus of Embodiment 1, wherein the renal denervation index comprises a scale between a lowest value and a highest value.
Embodiment 3. The apparatus of Embodiment 1, wherein calculating the value of the renal denervation index using the plurality of physiological metrics involves applying at least one of weights or scaling factors to the physiological metrics.
Embodiment 4. The apparatus of Embodiment 1, wherein calculating the value of the renal denervation index using the plurality of physiological metrics involves applying conditional categorization to at least some of the physiological metrics.
Embodiment 5. The apparatus of Embodiment 1, wherein calculating the value of the renal denervation index using the plurality of physiological metrics involves at least one physiological metric whose value varies with measurement location, such that the renal denervation index varies with the measurement location.
Embodiment 6. The apparatus of Embodiment 5, wherein the varying of the renal denervation index with location is used to select a treatment location.
Embodiment 7. The apparatus of Embodiment 1, wherein calculating the value of the renal denervation index using the plurality of physiological metrics involves at least one physiological metric whose value varies with measurement time, such that the renal denervation index varies with the measurement time.
Embodiment 8. The apparatus of Embodiment 6, wherein the varying of the renal denervation index with time is used to detect progress of a renal denervation treatment.
Embodiment 9. The apparatus of Embodiment 1, wherein calculating the value of the renal denervation index using the plurality of physiological metrics involves calculating at least one physiological metrics using at least two other physiological metrics of the plurality of physiological metrics.
Embodiment 10. The apparatus of Embodiment 1, wherein calculating the value of the renal denervation index using the plurality of physiological metrics involves a trained machine learning network.
Embodiment 11. The apparatus of Embodiment 1, wherein the plurality of physiological metrics includes at least one of imaging-derived measurements or invasive measurements.
Embodiment 12. The apparatus of Embodiment 1, wherein the plurality of physiological metrics includes at least one of height, weight, body-mass index (BMI), blood pressure (BP), ambulatory BP, mean BP, systolic BP, diastolic BP, heart rate (HR), resting HR, peak HR, mean HR, pulse-wave velocity, vascular resistance, renal vascular resistance, vascular flow rate, microvascular resistance, nerve conduction, renal nerve resting potential, glomerular filtration rate (GFR), aortic stiffness index (ASI), acute kidney injury classification (AKI, or concentrations of a biomarker molecule.
Embodiment 13. The apparatus of Embodiment 1, wherein the non-invasive measurement device comprises an X-ray, angiography, computer-aided tomography (CT), positron emission tomography (PET), magnetic resonance imaging (MRI), or ultrasound imaging device.
Embodiment 14. The apparatus of Embodiment 1, wherein the non-invasive measurement device comprises a blood pressure cuff, a pulse oximeter, an electrocardiogram (EKG) machine.
Embodiment 15. The apparatus of Embodiment 1, wherein the invasive measurement device comprises an intraluminal catheter or guidewire.
Embodiment 16. A method, comprising:
   obtaining, with a processor, values for a plurality of physiological metrics for a patient from at least one of an electronic health record database, a non-invasive measurement device, or an invasive measurement device;
   calculating, with the processor, a value of a renal denervation index using the plurality of physiological metrics such that the renal denervation index is itself not measured from the patient, wherein the value of the renal denervation index is representative of at least one of a suitability of the patient for a renal denervation treatment or an expected responsiveness of the patient to the renal denervation treatment; and
   outputting, to a display, a screen display based on the value of the renal denervation index.

## Claims

1. A processor configured to:
calculate a value of a renal denervation index using a plurality of physiological metrics for a patient from at least one of an electronic health record database, a non-invasive measurement device, or an invasive measurement device, such that the renal denervation index is itself not measured from the patient, wherein the value of the renal denervation index is representative of at least one of a suitability of the patient for a renal denervation treatment or an expected responsiveness of the patient to the renal denervation treatment; and
output, to a display, a screen display based on the value of the renal denervation index.

2. The processor of claim 1, wherein the renal denervation index comprises a scale between a lowest value and a highest value.

3. The processor of claim 1 or 2, wherein calculating the value of the renal denervation index using the plurality of physiological metrics involves applying at least one of weights or scaling factors to the physiological metrics.

4. The processor of any one of claims 1 to 3, wherein calculating the value of the renal denervation index using the plurality of physiological metrics involves applying conditional categorization to at least some of the physiological metrics.

5. The processor of any one of claims 1 to 4, wherein calculating the value of the renal denervation index using the plurality of physiological metrics involves at least one physiological metric whose value varies with measurement location, such that the renal denervation index varies with the measurement location, optionally, wherein the varying of the renal denervation index with location is used to select a treatment location.

6. The processor of any one of claims 1 to 5, wherein calculating the value of the renal denervation index using the plurality of physiological metrics involves at least one physiological metric whose value varies with measurement time, such that the renal denervation index varies with the measurement time, optionally, wherein the varying of the renal denervation index with time is used to detect progress of a renal denervation treatment.

7. The processor of any one of claims 1 to 6, wherein calculating the value of the renal denervation index using the plurality of physiological metrics involves calculating at least one physiological metrics using at least two other physiological metrics of the plurality of physiological metrics.

8. The processor of any one of claims 1 to 7, wherein calculating the value of the renal denervation index using the plurality of physiological metrics involves a trained machine learning network.

9. The processor of any one of the claims 1 to 8, wherein the plurality of physiological metrics includes at least one of imaging-derived measurements or invasive measurements.

10. The processor of any one of the claims 1 to 9, wherein the plurality of physiological metrics includes at least one of height, weight, body-mass index (BMI), blood pressure (BP), ambulatory BP, mean BP, systolic BP, diastolic BP, heart rate (HR), resting HR, peak HR, mean HR, pulse-wave velocity, vascular resistance, renal vascular resistance, vascular flow rate, microvascular resistance, nerve conduction, renal nerve resting potential, glomerular filtration rate (GFR), aortic stiffness index (ASI), acute kidney injury classification (AKI, or concentrations of a biomarker molecule.

11. The processor of any one of claim 1 to 10, wherein the non-invasive measurement device comprises at least one of: an X-ray, angiography, computer-aided tomography (CT), positron emission tomography (PET), magnetic resonance imaging (MRI), ultrasound imaging device, a blood pressure cuff, a pulse oximeter, an electrocardiogram (EKG) machine, or an intraluminal catheter or guidewire.

12. An apparatus comprising:
a processor of any one of claims 1 to 11; and
the non-invasive measurement device comprising at least one of: an X-ray, angiography, computer-aided tomography (CT), positron emission tomography (PET), magnetic resonance imaging (MRI), ultrasound imaging device, a blood pressure cuff, a pulse oximeter, an electrocardiogram (EKG) machine, or an intraluminal catheter or guidewire; and optionally, the display.

13. A method, comprising:
calculating, with the processor, a value of a renal denervation index using a plurality of physiological metrics for a patient from at least one of: an electronic health record database, a non-invasive measurement device, or an invasive measurement device such that the renal denervation index is itself not measured from the patient, wherein the value of the renal denervation index is representative of at least one of a suitability of the patient for a renal denervation treatment or an expected responsiveness of the patient to the renal denervation treatment; and
outputting, to a display, a screen display based on the value of the renal denervation index.

14. The method of claim 13, comprising:
obtaining, with the processor, values for a plurality of physiological metrics for a patient from at least one of an electronic health record database, a non-invasive measurement device, or an invasive measurement device.

15. A computer program, when executed by a processor, invokes execution of a computer program which, when executed, causes implementation of claim 13 or 14.
